# EUROPEAN PATENT SPECIFICATION

(11) **EP 4 079 227 B1**
(45) Date of publication and mention of the grant of the patent: **04.06.2025**
(21) Application number: 21170167.7
(22) Date of filing: 23.04.2021
(51) Int. Cl.: A61B 6/03, A61B 6/00

(54) **ADAPTION OF A MEDICAL IMAGING PROCESS TO AN INDIVIDUAL RESPIRATION BEHAVIOUR OF A PATIENT**
ANPASSUNG EINES MEDIZINISCHEN BILDGEBUNGSVERFAHRENS AN EIN INDIVIDUELLES ATMUNGSVERHALTEN EINES PATIENTEN
ADAPTATION D'UN PROCESSUS D'IMAGERIE MÉDICALE AU COMPORTEMENT RESPIRATOIRE INDIVIDUEL D'UN PATIENT

(43) Date of publication of application: 26.10.2022
(73) Proprietor: Siemens Healthineers AG, 91301 Forchheim (DE)
(72) Inventor: Baer-Beck, Matthias, 91080 Spardorf (DE); Hofmann, Christian, 91052 Erlangen (DE)
(74) Representative: Siemens Healthineers Patent Attorneys

(56) References cited:
- US-A1- 2005 113 673
- US-A1- 2007 286 331
- US-A1- 2013 211 236
- US-A1- 2017 251 949

## Description

The invention relates to a method of adaption of a medical imaging process to an individual respiration behaviour of a patient. The invention also relates to an adaption device. Further, the invention relates to a medical imaging system.

Respiratory information collected by a so-called "respiratory surrogate" is used until now only in dedicated 4D respiratory scan modes and protocols of CT scan systems. Here the respiratory surrogate is used to determine the respiratory phase of a patient either during the scan in order to enable phase correlated scanning or after the scan in order to perform a phase correlated reconstruction of image data based on the scanned raw data.

US 2013/211236 A1, US 2017/251949 A1, US 2005/113673 A1 and US 2007/286331 A1 are to be mentioned as prior art.

In standard and non-respiratory scans and reconstructions a respiratory surrogate is not recorded at all. The reason for this is that up to now a respiratory surrogate was recorded by an external measurement system, which is quite complicated and complex to attach to the patient. Currently, a new radar-based system for the recording of the respiratory surrogate is being developed. This radar-based system is integrated into a patient table of a CT scan system and is therefore always available without any complicated and time consuming installation process. This new system for the recording of the respiratory surrogate offers the possibility to make use of the information about the breathing cycle and breathing properties of a patient even in non-respiratory correlated scan processes in order to make patient specific adjustments to the breathing commands before and during the scan.

Breathing commands are contained in almost any CT protocol. Typically, those commands are given right before or during the scan in order to give an advice to the patient how to control his breathing in order to avoid artifacts in the reconstructed images. Currently, the commands are typically pre-recorded speech commands, which can be recorded and stored by the clinical users of the CT scan units. Typically, every clinical imaging task, for example cardiac, dual energy, standard contrast and non-contrast exams have their dedicated commands, which are pre-recorded and stored in specific protocols by the clinical users according to their needs and clinical practice. The drawback of those pre-recorded commands is that they do not take into account the breathing properties of the current patient and that they cannot adapt to the current breathing status of the patient. For example there is no way to detect whether the patient is following the commands or not, which can in the worst case yield to non-diagnostic images and the need for a re-scan.

Hence, a problem underlying to the invention is to improve the effectivity of a CT scan process, which is influenced by a respiratory movement of a patient.

That problem is solved by a method of adaption of a medical imaging process to an individual respiration behaviour of a patient according to claim 1, by an adaption device according to claim 12 and by a medical imaging system according to claim 13.

According to the method of adaption of a medical imaging process, the medical imaging process being a CT scan process, to an individual respiration behaviour of a patient, it is proposed to use the monitoring of a respiratory surrogate to optimize the breathing guidance prior and during scanning and to guide the clinical user after the scan about the quality of the scan as long as the patient is still on the table. The method comprises the steps of recording the respiratory movement of a patient by monitoring a respiratory surrogate and adapting the medical imaging process based on the recorded respiratory movement of the patient, wherein the adaption is performed in the scan phase of the medical imaging process.

During the scan phase, it is determined whether the patient holds his breath properly, and in case the patient does not hold his breath properly, the scan process is dynamically reparametrized by increasing the pitch.

A surrogate has to be understood as a physiological process, which can be detected using sensor technology and which is correlated to another physiological process to be monitored. Hence, the actual interesting physiological process, for example the movement of an internal organ or the movement i.e. the change of a position of a predetermined internal examination area, can be determined based on the observation of the breathing movement of a patient, by correlating the breathing movement with the movement of the interesting internal portion. In particular, the respiratory surrogate represents the respiratory movement of a patient. The information about the respiratory movement can be used as feedback information for controlling breathing commands, which are harmonized with recording time intervals of the medical imaging process such that the imaging is not disturbed by the respiratory movement of the patient. Advantageously, the quality of medical images can be improved and the operating expense and time for wasted scan processes can be reduced. Hence, the medical imaging resources can be used more effectively. Further, the comfort for the patient is increased, since the number of additional scan attempts and the time for these attempts can be reduced. Furthermore, the radiation exposure due to additional scan attempts can also be reduced.

The adaption device according to the invention, preferably for a CT system, is configured for carrying out the method according to the invention and comprises a monitoring unit for recording the respiratory movement of the patient by monitoring the respiratory surrogate and an adaption unit for adapting the imaging process based on the recorded respiratory movement of the patient. The adaption device shares the advantages of the method according to the invention.

The medical imaging system is a CT system and comprises a scan unit for carrying out a medical imaging from a patient and an adaption device according to the invention for adapting the medical imaging of the scan unit to a recorded respiratory movement of the patient. Further, the medical imaging system includes a sensor device, for example a radar sensor, for acquiring sensor data concerning the respiratory movement of the patient. The medical imaging system shares the advantages of the method according to the invention.

The essential components of the scan adaption device according to the invention can for the most part be designed in the form of software components and if required, by further adding some hardware as for example a respiratory movement sensor like a radar sensor. This applies in particular to the monitoring unit and the adaption unit of the scan adaption device but also parts of the input interfaces. In principle, however, some of these components can be implemented also in the form of software-supported hardware, for example FPGAs or the like, especially when it comes to particularly fast calculations. Likewise, the required interfaces, for example if it is only a matter of transferring data from other software components, can be designed as software interfaces. However, they can also be designed as hardware-based interfaces that are controlled by suitable software. Furthermore, some parts of the above-mentioned components may be distributed and stored in a local or regional or global network or a combination of a network and software, in particular a cloud system.

A largely software-based implementation has the advantage that medical imaging systems that have already been used, can easily be retrofitted by a software update in order to work in the manner according to the invention. In this respect, the object is also achieved by a corresponding computer program product with a computer program that can be loaded directly into a memory device of for example a control device of a medical imaging system, with program sections, in order to carry out all steps of the method according to the invention, if the program is executed in the medical imaging system, in particular the control device. In addition to the computer program, such a computer program product may contain additional components such as a documentation and/ or additional components, including hardware components such as hardware keys (dongles etc.) for using the software.

For transport to the medical imaging system and/ or for storage on or in the medical imaging system, a computer-readable medium, for example a memory stick, a hard disk or some other transportable or permanently installed data carrier is used on which the program sections of the computer program that can be read in and executed by a computer unit of the medical imaging system are stored. The computer unit can comprise for example, one or more cooperating microprocessors or the like used for this purpose.

The dependent claims and the following description each contain particularly advantageous embodiments and developments of the invention. In particular, the claims of one claim category can also be further developed analogously to the dependent claims of another claim category. In addition, within the scope of the invention, the various features of different exemplary embodiments and claims can also be combined to form new exemplary embodiments.

In a variant of the method according to the invention the adaption is performed in at least one of the following phases of the medical imaging process:
- the pre-scan phase,
- the after-scan phase.

Advantageously, the information of the individual breathing behaviour of a patient can be used for the preparation of the scan process, as real time information for a dynamic adaption of the scan process during the actual scan process and as information for the steps after having finished the scan process like the reconstruction of image data or a repetition of the scan in case it has to be expected that the image quality of reconstructed image data would be not sufficient.

In a further variant of the method according to the invention during the pre-scan phase the adaption comprises the substeps of training the patient to breathing optimally for a given scan mode or task by taking into account the patients individual breathing behaviour for the given scan mode or task. Advantageously the recorded breathing behaviour of a patient can be used as feedback information for a feedback loop for training the patient for an individual scan mode or task.

In a further variant of the method according to the invention, during the pre-scan phase the adaption comprises determining patient depended, individualized optimal timed breathing commands. For example, the reaction time of a patient can be determined based on the recorded breathing behaviour of the patient. Hence, the breathing commands can be scheduled such that the determined delay of the reaction of the patient is compensated.

Also the optimal point in time for the scan to start can be determined based on the analysis of the patient breathing pattern. The scan can be delayed, if the patient is currently in an unsuitable breathing phase to follow the given breathing command in time. That means the breathing command will be played when the time point is well suited for the patients current breathing phase.

In a further variant of the method according to the invention, during the pre-scan phase the scan process is parametrized based on the breathing behaviour. It is determined, if the patient is able to hold his breath for performing the scan in the current configuration. If not, the control unit of the medical imaging system can give a hint to decrease the scan time, e.g. increase the pitch or switch to another scan mode, e.g. from standard spirals to a high pitch flash scan,

if the medical imaging system provides that option. In general, these switches can also be done automatically, if local standards and regulations allow for automatic adaptions of the scan parameterization.

In a variant of the method according to the invention, during the scan phase, it is detected, if the patient holds his breath properly, and in case the patient does not hold his breath properly, a command is played to remind and motivate the patient to further hold the breath. Advantageously, a feedback of the behaviour of the patient can be used to try to influence the breathing behaviour of the patient in real time to save a current scan operation in case the patient does not exactly follow the breathing commands.

According to the invention, the scan process is dynamically reparametrized by increasing the pitch, if the patient seems to be not able to hold the breath properly. In that case, in particular if the patient does not improve his breathing behaviour, although he has been admonished to do so, the scan process can be altered such that the speed of recordation of raw data is increased such that the shortened breath-hold time of the patient can be tolerated.

In a further variant of the method according to the invention, during the scan phase, the scan process is aborted in an early stage, if it is detected based on the recorded breathing behaviour that the scan results are likely insufficient and a rescan cannot be avoided. Advantageously, scan resources and time resources can be saved, if it is very likely that a current scan process would lead to a medical image with insufficient image quality.

Further, during the scan phase, it can be detected, if the patient holds his breath properly, and in case the patient does not hold his breath properly, the scan process can be stopped and a pause of the scan process can be carried out at a relevant z-position, where the patient is allowed to stop holding the breath. Under the z-position, it has to be understood the coordinate of the presently recorded slice in **z-**direction, which is the direction of the system axis of a medical imaging system, the medical imaging system being a CT system. In that case, after the pause, the patient is instructed to perform breath-hold again and the scan process is continued at the z-position, where the scan process was stopped or at a position, where the patient still hold his breath properly. Advantageously, a current scan process can be saved although the patient is transiently not able to follow the breathing commands of the medical imaging system properly.

In a further variant of the method according to the invention, in the after-scan phase the individual breathing behaviour of the patient is analysed based on a recorded breathing curve and in case it is detected that the patient did not follow the breathing commands properly during the scan phase, a re-scan is recommended, if severe artifacts are expected. Advantageously, the decision, if the scan has to be repeated or not can be automatically carried out based on the feedback information about the patients breathing behaviour. In that case, a reconstruction based on deteriorated raw data can be dismissed and hence, time resources and medical examination capacities can be saved.

Furthermore, in a variant of the method according to the invention, in at least one of the pre-scan phase, the scan phase and the after-scan-phase, it is automatically determined based on the recorded breathing behaviour how to adapt the medical imaging process. That means that it is decided which measurement or which combination of the above-mentioned measurements for an improvement of the scan process is carried out based on the information of a respiratory surrogate. The analysis for that decision can be implemented using classical signal processing approaches or by the means of deep learning-based algorithms. For example, the analysis of the respiratory curve can be done by some kind of neural network.

In a more detailed described variant of the adaption device, the adaption unit comprises a breathing training unit for emitting breathing commands based on the recorded breathing data and the determined breathing curve. Furthermore, the adaption unit can optionally include a starting point determination unit for setting the starting time point of a scan and a breathing command based on the recorded breathing curve. The adaption unit can also include a parametrization unit for adapting scan parameters and selecting an adapted scan protocol for a recorded individual breathing curve. The parametrization unit may also be able to reparametrize a scan process in real time during a running scan process. Moreover, the adaption unit may include an interrupting unit for stopping the scan process, if it has been detected that the breathing behaviour of the patient deteriorates during the scan process. The interrupting unit stops the scan process by emitting an interruption command to the scan unit of the medical imaging system and may for example determine a z-position based on the recorded breathing curve, at which the scan process can be resumed after the patient has recovered. Furthermore, the adaption unit may additionally comprise a recommendation unit for emitting a recommendation of abortion or rescanning in case severe artifacts are to be expected based on the recorded breathing curve. Hence, the adaption device according to that variant may comprise a multiplicity of functionalities for adapting a scan process to an individual breathing behaviour of a patient for improving image quality and for saving scan resources in case it is early detected that a scan process should be aborted due to a lack of quality of the recorded images.

The adaption device according to the invention may be part of a control unit, which is for example part of a medical imaging system, the medical imaging system being a computed tomography system. The control unit can optionally comprise a data storage unit, which stores scan protocols, which can be used for a particular scan mode. Such a scan protocol is transmitted to a driving unit. The driving unit, which may be also part of the control unit, generates control instructions for controlling a scan process. The control instructions are sent to the scan unit of the medical imaging system. The control unit may also include a reception unit, which receives raw data from the scan unit during a scan process and which also receives breathing data from a sensor system, for example a radar sensor system, which may be installed in a patient table of the medical imaging system, and monitors the breathing movement of the patient. Then, the breathing data are transmitted to an adaption device according to a variant of the invention, which may be also part of the control unit. The adaption device may modify the currently used scan protocol and may then transmit a modified scan protocol to the driving unit. The scan adaption device can also directly transmit instructions to the driving unit, for example a stop instruction, i.e. an interruption command or a start instruction or movement instruction for the patient table or the movable part of the scan unit of the medical imaging system. The scan adaption device can also emit a modified breathing command to the driving unit, which is sent to the scan unit and is emitted by an audio system of the scan unit such that the patient can follow the modified breathing command. Further, the scan adaption device may further send a breathing curve to a reconstruction unit, which may be also part of the control unit and reconstructs image data based on the received raw data and the breathing curve. Furthermore, the scan adaption device can emit a recommendation to the user for abortion or rescanning, in case the breathing curve leads to the conclusion that the image quality of the reconstructed medical images would likely be not high enough for a particular application of the medical images generated by the scan process.

The invention is explained below with reference to the figures enclosed once again. The same components are provided with identical reference numbers in the various figures. The figures are usually not to scale.
FIG 1 shows a CT-system according to an embodiment of the invention,
FIG 2 shows a flow chart diagram illustrating the method of adaption of a medical imaging process to an individual respiration behaviour of a patient according to an embodiment of the invention,
FIG 3 shows a flow chart diagram illustrating the adaption of the medical imaging process in the pre-scan phase,
FIG 4 shows a flow chart diagram illustrating the adaption of the medical imaging process during the scan phase,
FIG 5 shows a flow chart diagram illustrating the adaption of the medical imaging process in the after-scan phase,
FIG 6 shows a schematic view onto an adaption device according to an embodiment of the invention,
FIG 7 shows a schematic view onto a control unit of a CT system, comprising the adaption device shown in FIG 6.

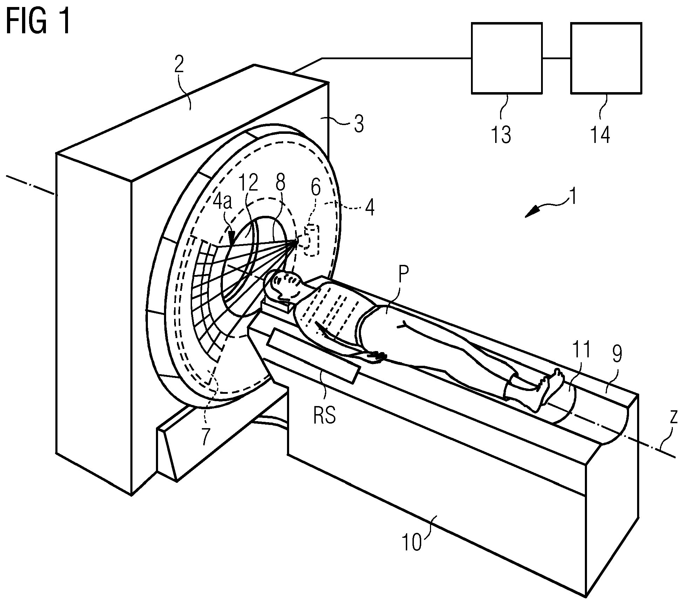

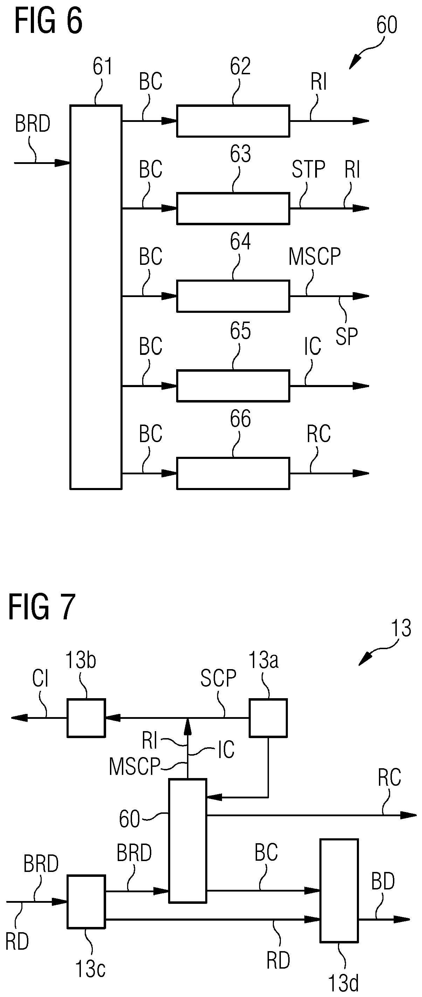

FIG 1 shows s schematic representation of a computed tomography system 1 comprising an adaption device according to an embodiment of the invention as later discussed in detail in context with FIG 6 and FIG 7 and which may be included by a computer 13 of the computed tomography system 1. The arrangement of the computed tomography system 1 comprises a gantry also called as scan unit 2 with a stationary part 3, also referred to as a gantry frame, and with a part 4, which can be rotated about a system axis z, also referred to as a rotor or drum. The rotating part 4 has an imaging system (X-ray system) 4a, which comprises an X-ray source 6 and an X-ray detector 7, which are arranged on the rotating part 4 opposite one another. The X-ray source 6 and the X-ray detector 7 form the imaging system 4a. When the computed tomography system 1 is in operation, the X-ray source 6 emits X-rays 8 in the direction of the X-ray detector 7, penetrates a measurement object P, for example a patient P, and is transmitted by the X-ray detector 7 in the form of measurement data or measurement signals recorded.

In FIG 1, a patient table 9 for positioning the patient P can also be seen. The patient table 9 comprises a bed base 10, on which a patient support plate 11, which is provided for actually positioning the patient P, is arranged. The patient support plate 11 can be adjusted relative to the bed base 10 in the direction of the system axis z, i.e. in the z direction, so that it enters an opening 12 such that the patient P can be introduced into the opening 12 of the scan unit 2 for recording X-ray projections from the patient P. A computational processing of the X-ray projections recorded with the imaging system 4a or the reconstruction of sectional images, 3D images or a 3D data set based on the measurement data or measurement signals of the X-ray projections is carried out in an image computer 13 of the computed tomography device 1, wherein the sectional images or 3D images can be displayed on a display device 14. The image computer 13 can also be designed as a control unit for controlling an imaging process and for controlling the scan unit 2 and in particular the imaging system 4a of the scan unit 2. In FIG 1, also a sensor device, which works as a surrogate monitoring unit RS, is depicted. The surrogate monitoring unit RS emits radar waves (symbolised as dashed lines in FIG 1) to the area to be scanned, which is the breast of the patient P.

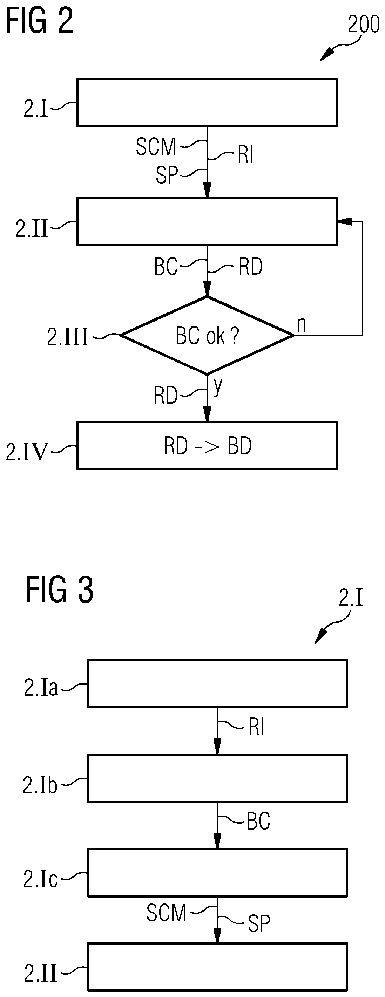

FIG 2 shows a flow chart diagram 200, which illustrates the method of adaption of a medical imaging process to an individual respiration behaviour of a patient P according to an embodiment of the invention.

In step 2.I a pre-scan adaption is carried out. Step 2.I comprises a breathing training of a patient P, an adaption of breathing commands RI, a selection of an appropriate scan mode SCM and an adaption of parameters SP of the selected scan mode SCM based on the patient's individual breathing behaviour. Further details of step 2.I are described in context with FIG 3.

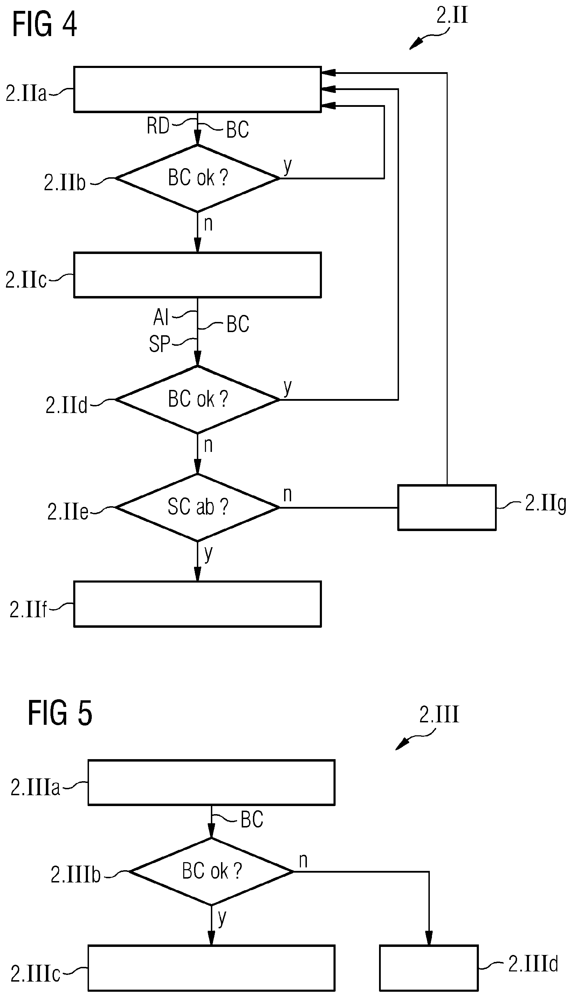

In step 2.II a during-scan adaption is carried out, wherein raw data RD are acquired from the patient. Step 2.II comprises a real time adaption of the scan process during the scanning. As explained in detail in context with FIG 4, the scan process can be dynamically reparametrized, the scan can be aborted in an early stage or the scan can be interrupted, until the patient P has recovered and then the scan process can be resumed at a z-position, at or after which the breathing curve BC shows an aberration of the breathing behaviour of the patient P from a predetermined breathing curve.

In step 2.III an after-scan adaption of the imaging process is carried out and it is determined based on the recorded breathing curve BC, whether the patient P followed the breathing commands properly during the scan time or not. If that is the case, which is symbolized in FIG 2 with "y", step 2.IV can be carried out, wherein a reconstruction based on the recorded raw data RD of the scan is performed and image data BD are reconstructed. If it is determined in step 2.III that the patient did not follow properly enough to the breathing instructions, which is symbolized in FIG 2 with "n", it is decided that the image data BD have to be discarded and the scan process has to be repeated and the method goes further with step 2.II. Further details concerning step 2.III are discussed in context with FIG 5.

In FIG 3 a flow chart diagram of step 2.I is illustrated. In sub step 2.Ia, a breathing training is performed, wherein a patient P gets some instructions RI for the point of time, when he should hold his breath and for the point of time, when he can proceed with breathing. The training process comprises a feedback loop. That means that in sub step 2.Ib the patient P is monitored and a reaction of the patient P to an instruction RI is recorded. Particularly, a breathing curve BC is recorded. The training can also be used for taking into account the patient's individual breathing behaviour. For example, if the patient P needs some time for reacting to an instruction RI, the individual reaction time of the patient P can be considered for setting the starting time point of a scan process. In sub step 2.Ic based on the recorded breathing curve BC, a scan mode SCM or an adapted set SP of parameters for a scan process is determined. Thereafter, the scan process is carried out with step 2.II, which is described in context with FIG 4 in detail.

In FIG 4 a flow chart diagram is illustrated for a detailed description of step 2.II. In sub step 2.IIa, the actual scan process is started and some raw data RD from a scan area of the patient P are recorded. At the same time, a breathing curve BC, describing the breathing behaviour of the patient P, is recorded. At sub step 2.IIb it is determined, if the breathing curve BC is conform with a predetermined breathing curve. If that is the case, which is represented in FIG 4 with "y", the scan process can be continued with sub step 2.IIa. If the breathing curve BC deviates from the predetermined breathing curve, which is represented with "n" in FIG 4, in sub step 2.IIc a command AI is played out to remind and motivate the patient P to further hold the breath. If it is helpful, the scan process can be dynamically reparametrized, by for example increasing the pitch, if the patient P seems to be not able to hold the breath long enough, or amending some other scan parameters SP. In sub step 2.IId it is determined, if the recorded breathing curve BC is conform with a possibly amended predetermined breathing curve, which, where appropriate, has been adapted to the amended parameters SP. If that is the case, which is symbolized with "y" in FIG 4, the scan process proceeds with sub step 2.IIa. If the recorded breathing BC still deviates from the amended breathing curve, which is symbolized with "n" in FIG 4, the method goes further with sub step 2.IIe. In sub step 2.IIe it is determined, if the scan SC has to be aborted. An abortion can be reasonable, for example in an early stage of a scan process, if it is obvious that the scan result is insufficient and a rescan cannot be avoided. If that is the case, which is symbolized with "y" in FIG 4, the scan process ends with sub step 2.IIf. In case the scan SC has not been finished, which is symbolized with "n" in FIG 4, in sub step 2.IIg the scan SC is stopped such that the patient P can recover. After a recovering pause, a z-position is determined, at which the measured breathing curve BC was conform enough with a predetermined breathing curve. Then the scan SC is resumed with sub step 2.IIa at the determined z-position. It is confirmed that most of the sub steps of step 2.II, for example sub step 2.IIe or sub step 2.IIg are optional and can be leaved out in other embodiments of the invention or arranged in another combination as described in context with FIG 4.

In FIG 5 a flow chart is illustrated, which describes step 2.III of the adaption method visualized in FIG 2 in detail.

In sub step 2.IIIa, after the actual scan process, the recorded breathing curve BC is analysed and compared with a predetermined breathing curve. In sub step 2.IIIb, it is determined, if the recorded breathing curve BC is conform enough with the predetermined breathing curve. If that is the case, which is symbolised with "y" in FIG 5, the process ends with sub step 2.IIIc, wherein the reconstruction of recorded raw data RD is carried out. In case the recorded breathing curve BC is not conform enough with the predetermined breathing curve, which is symbolised with "n" in FIG 5, the scan process ends with sub step 2.IIId, wherein the recorded raw data RD are dismissed and a new scan is recommended.

In FIG 6 an adaption device 60 is schematically illustrated. The adaption device 60 comprises a monitoring unit 61 for recording breathing data BRD of a respiratory movement of a patient and generating a breathing curve BC based on the recorded breathing data BRD. Further, the adaption device 60 also comprises a breathing training unit 62 for emitting breathing commands RI based on the recorded breathing data BRD and the determined breathing curve BC. Furthermore, the adaption device 60 includes a starting point determination unit 63 for setting the starting time point STP of a scan and a breathing command RI based on the determined breathing curve BC.

The adaption device 60 also includes a parametrization unit 64 for adapting scan parameters SP and selecting an adapted scan protocol MSCP for a determined individual breathing curve BC. The parametrization unit 64 is also able to reparametrize a scan process in real time during a running scan process. Moreover, the adaption device 60 has an interrupting unit 65 for stopping the scan process, if it has been detected that the breathing behaviour of the patient deteriorates during the scan process. The interrupting unit 65 stops the scan process by emitting an interruption command IC to the scan unit 2 and determines a z-position based on the recorded and determined breathing curve BC, at which the scan process can be resumed after the patient P has recovered. Furthermore, the adaption device 60 comprises a recommendation unit 66 for emitting a recommendation RC of abortion or rescanning in case severe artifacts are to be expected based on the breathing curve BC.

In FIG 7, a control unit 13 is illustrated, as it is for example part of the computed tomography system 1 shown in FIG 1. The control unit 13 comprises a data storage unit 13a, which stores scan protocols SCP, which can be used for a particular scan mode. Such a scan protocol SCP is transmitted to a driving unit 13b. The driving unit 13b, which is also part of the control unit 13, generates control instructions CI for controlling a scan process. The control instructions CI are sent to the scan unit 2 (shown for example in FIG 1). The control unit 13 also includes a reception unit 13c, which receives raw data RD from the scan unit 2 during a scan process and which also receives breathing data BRD from a radar sensor system RS (as depicted in FIG 1), which is installed in the patient table 9 of the computed tomography system 1, and monitors the breathing movement of the patient P. The breathing data BRD are transmitted to an adaption device 60, which is also part of the control unit 13 and is composed of the parts described in context with FIG 6. The adaption device 60 modifies the currently used scan protocol SCP and transmits a modified scan protocol MSCP to the driving unit 13b. The scan adaption device 60 can also directly transmit instructions to the driving unit 13b, for example a stop instruction, i.e. an interruption command IC or a start instruction or movement instruction for the patient table 9 or the movable part 4a of the scan unit 2. The scan adaption device 60 can also emit a modified breathing command RI to the driving unit 13b, which is sent to the scan unit 2 and emitted by an audio system of the scan unit 2 such that the patient P can follow the modified breathing command RI. Further, the scan adaption device 60 also sends a breathing curve BC to a reconstruction unit 13d, which reconstructs image data BD based on the received raw data RD and the breathing curve BC. Furthermore, the scan adaption device 60 emits a recommendation RC to the user for abortion or rescanning, in case the breathing curve BC leads to the conclusion that the image quality of the reconstructed medical images would likely be not high enough for a particular application of the medical images generated by the scan process.

Further, the use of the undefined article "a" or "one" does not exclude that the referred features can also be present several times. Likewise, the term "unit" or "device" does not exclude that it consists of several components, which may also be spatially distributed.

## Claims

1. Method of adaption of a medical imaging process to an individual respiration behaviour of a patient (P), the medical imaging process being a CT scan process, the method comprising the steps of:
- recording a respiratory movement (BRD) of a patient (P) by monitoring a respiratory surrogate,
- adapting the medical imaging process based on the recorded respiratory movement (BRD) of the patient (P), wherein the adaption is performed in the scan phase of the medical imaging process,
- **characterised in that** during the scan phase, it is determined whether the patient (P) holds his breath properly, and in case the patient (P) does not hold his breath properly, the scan process is dynamically reparametrized by increasing the pitch.

2. Method according to claim 1, wherein the adaption is additionally performed in at least one of the following phases of the medical imaging process:
- the pre-scan phase,
- the after-scan phase.

3. Method according to claim 2, wherein during the pre-scan phase the adaption comprises training the patient (P) to breath optimally for a given scan mode (SCM) or task by taking into account individual breathing behaviour (BRD) of the patient (P) for the given scan mode (SCM) or task.

4. Method according to claim 2 or 3, wherein during the pre-scan phase the adaption comprises determining patient depended, individualized optimal timed breathing commands (RI).

5. Method according to claim 4, wherein during the pre-scan phase the optimal point of time (STP) for the scan (SC) to start is determined based on an analysis of a detected pattern of the breathing behaviour (BRD) of the patient (P).

6. Method according to anyone of the claims 2 to 5, wherein during the pre-scan phase the scan process (SC) is parametrized based on the breathing behaviour (BRD) of the patient (P).

7. Method according to anyone of the claims 1 to 6, wherein in the case the patient (P) does not hold his breath properly,
- a command (RI) is played out to remind and motivate the patient (P) to further hold the breath.

8. Method according to anyone of the claims 1 to 7, wherein during the scan phase, the scan process is aborted, preferably in an early stage, if it has been detected based on the recorded breathing behaviour (BRD) that the scan results are likely insufficient and a rescan cannot be avoided.

9. Method according to anyone of the claims 1 to 7, wherein during the scan phase, it is determined, if the patient (P) holds his breath properly, and in case the patient (P) does not hold his breath properly,
- the scan process (SC) is stopped and a pause of the scanning is carried out at a z-position, where the patient (P) is allowed to stop holding the breath,
- after the pause, the patient (P) is instructed to perform breath-hold again, and
- the scan process (SC) is continued at the z-position, where the scan process (SC) was stopped or at a z-position, where the patient (P) still hold his breath properly.

10. Method according to anyone of the claims 2 to 9, wherein during the after-scan phase, the individual breathing behaviour of the patient (P) is analysed based on a recorded breathing curve (BC) and in case it is detected that the patient (P) did not follow the breathing commands (RI) properly during the scan phase and if severe artifacts are expected, a re-scan is recommended.

11. Method according to anyone of the claims 2 to 9, wherein in at least one of the pre-scan phase, the scan phase or the after-scan-phase, it is automatically determined based on the recorded breathing behaviour (BRD) how to adapt the scan medical imaging process to the recorded breathing behaviour (BRD) .

12. Adaption device (60), configured for carrying out the method according to anyone of the claims 1 to 11 and comprising:
- a monitoring unit (61) for recording the respiratory movement (BRD) of the patient (P) by monitoring the respiratory surrogate,
- an adaption unit (62, 63, 64, 65, 66) for adapting the medical imaging process based on the recorded respiratory movement (BRD) of the patient (P).

13. Medical imaging system (1), the medical imaging system (1) being a CT system and comprising:
- a scan unit (2) for carrying out a medical imaging from a patient (P),
- an adaption device (60) according to claim 12 for adapting the medical imaging of the scan unit (2) to a recorded respiratory movement (BRD) of the patient (P),
- a sensor device (RS) for acquiring sensor data concerning the respiratory movement (BRD) of the patient (P).

14. Computer program product with a computer program, which can be loaded directly into a memory device (13a) of a medical imaging system (1), with program sections to perform all the steps of anyone of the claims 1 to 11, when the computer program is carried out in the medical imaging system (1).

15. Computer readable medium, on which program sections that can be read in and executed by a computer unit (13) are stored in order to carry out all steps of the methods according to anyone of the claims 1 to 11, when the program sections are executed by the computer unit (13).

## Patentansprüche

1. Verfahren zur Anpassung eines medizinischen Bildgebungsprozesses an ein individuelles Atemverhalten eines Patienten (P), wobei der medizinische Bildgebungsprozess ein CT-Scan-Prozess ist, wobei das Verfahren die folgenden Schritte umfasst:
- Aufzeichnen einer Atembewegung (BRD) eines Patienten (P) durch Überwachen eines Atemsurrogats;
- Anpassen des medizinischen Bildgebungsprozesses basierend auf der aufgezeichneten Atembewegung (BRD) des Patienten (P), wobei die Anpassung in der Scan-Phase des medizinischen Bildgebungsprozesses durchgeführt wird,
- **dadurch gekennzeichnet, dass** während der Scan-Phase bestimmt wird, ob der Patient (P) seinen Atem richtig anhält, und, falls der Patient (P) seinen Atem nicht richtig anhält, der Scan-Prozess durch Erhöhen des Pitchs dynamisch neu parametriert wird.

2. Verfahren nach Anspruch 1, wobei die Anpassung zusätzlich in mindestens einer der folgenden Phasen des medizinischen Bildgebungsprozesses durchgeführt wird:
- Phase vor dem Scannen,
- Phase nach dem Scannen.

3. Verfahren nach Anspruch 2, wobei während der Phase vor dem Scannen die Anpassung Trainieren des Patienten (P) zur optimalen Atmung für einen gegebenen Scan-Modus (SCM) oder eine gegebene Aufgabe umfasst, indem das individuelle Atemverhalten (BRD) des Patienten (P) für den gegebenen Scan-Modus (SCM) oder die gegebene Aufgabe berücksichtigt wird.

4. Verfahren nach Anspruch 2 oder 3, wobei während der Phase vor dem Scannen die Anpassung Bestimmen patientenabhängiger, individualisierter optimaler zeitlich festgelegter Atembefehle (RI) umfasst.

5. Verfahren nach Anspruch 4, wobei während der Phase vor dem Scannen der optimale Zeitpunkt (STP) für den Start des Scans (SC) basierend auf einer Analyse eines erkannten Musters des Atemverhaltens (BRD) des Patienten (P) bestimmt wird.

6. Verfahren nach einem der Ansprüche 2 bis 5, wobei während der Phase vor dem Scannen der Scan-Prozess (SC) basierend auf dem Atemverhalten (BRD) des Patienten (P) parametriert wird.

7. Verfahren nach einem der Ansprüche 1 bis 6, wobei in dem Fall, dass der Patient (P) seinen Atem nicht richtig anhält,
- ein Befehl (RI) ausgegeben wird, um den Patienten (P) daran zu erinnern und zu motivieren, den Atem weiter anzuhalten.

8. Verfahren nach einem der Ansprüche 1 bis 7, wobei während der Scan-Phase der Scan-Prozess vorzugsweise in einem frühen Stadium abgebrochen wird, falls basierend auf dem aufgezeichneten Atemverhalten (BRD) erkannt wurde, dass die Scan-Ergebnisse wahrscheinlich unzureichend sind und ein erneuter Scan nicht vermieden werden kann.

9. Verfahren nach einem der Ansprüche 1 bis 7, wobei während der Scan-Phase bestimmt wird, ob der Patient (P) seinen Atem richtig anhält, und in dem Fall, dass der Patient (P) seinen Atem nicht richtig hält,
- der Scan-Prozess (SC) gestoppt und eine Pause des Scannens an einer z-Position erfolgt, an der der Patient (P) das Anhalten des Atems stoppen darf;
- nach der Pause der Patient (P) angewiesen wird, den Atem erneut anzuhalten, und
- der Scan-Prozess (SC) an der z-Position, an der der Scan-Prozess (SC) gestoppt wurde, oder an einer z-Position, an der der Patient (P) seinen Atem noch richtig anhält, fortgesetzt wird.

10. Verfahren nach einem der Ansprüche 2 bis 9, wobei während der Phase nach dem Scannen das individuelle Atemverhalten des Patienten (P) basierend auf einer aufgezeichneten Atemkurve (BC) analysiert wird und in dem Fall, dass erkannt wird, dass der Patient (P) die Atembefehle (RI) während der Scan-Phase nicht richtig befolgt hat, und, falls schwere Artefakte erwartet werden, ein erneuter Scan empfohlen wird.

11. Verfahren nach einem der Ansprüche 2 bis 9, wobei in mindestens einer von der Phase vor dem Scannen, der Scan-Phase oder der Phase nach dem Scannen basierend auf dem aufgezeichneten Atemverhalten (BRD) automatisch bestimmt wird, wie der medizinische Scan-Bildgebungsprozess an das aufgezeichnete Atemverhalten (BRD) angepasst werden soll.

12. Anpassungsvorrichtung (60), die zum Durchführen des Verfahrens nach einem der Ansprüche 1 bis 11 ausgelegt ist und Folgendes umfasst:
- eine Überwachungseinheit (61) zum Aufzeichnen der Atembewegung (BRD) des Patienten (P) durch Überwachen des Atemsurrogats,
- eine Anpassungseinheit (62, 63, 64, 65, 66) zum Anpassen des CT-Bildgebungsprozesses basierend auf der aufgezeichneten Atembewegung (BRD) des Patienten (P).

13. Medizinisches Bildgebungssystem (1), wobei das medizinische Bildgebungssystem (1) ein CT-System ist und Folgendes umfasst:
- eine Scan-Einheit (2) zum Durchführen einer medizinischen Bildgebung von einem Patienten (P),
- eine Anpassungsvorrichtung (60) nach Anspruch 12 zum Anpassen der medizinischen Bildgebung der Scan-Einheit (2) an eine aufgezeichnete Atembewegung des Patienten (P).
- eine Sensorvorrichtung (RS) zum Erfassen von Sensordaten bezüglich der Atembewegung (BRD) des Patienten (P).

14. Computerprogrammprodukt mit einem Computerprogramm, das direkt in eine Speichervorrichtung (13a) eines medizinischen Bildgebungssystems (1) geladen werden kann, mit Programmabschnitten zum Ausführen aller Schritte nach einem der Ansprüche 1 bis 11, wenn das Computerprogramm in dem medizinischen Bildgebungssystem (1) ausgeführt wird.

15. Computerlesbares Medium, auf dem Programmabschnitte gespeichert sind, die von einer Computereinheit (13) eingelesen und ausgeführt werden können, um alle Schritte der Verfahren nach einem der Ansprüche 1 bis 11 auszuführen, wenn die Programmabschnitte von der Computereinheit (13) ausgeführt werden.

## Revendications

1. Procédé d'adaptation d'un processus d'imagerie médicale à un comportement respiratoire individuel d'un patient (P), le processus d'imagerie médicale étant un processus de scan CT, le procédé comprenant les stades de :
- enregistrement d'un mouvement (BRD) respiratoire d'un patient (P) en contrôlant un substitut respiratoire,
- adaptation du processus d'imagerie médicale sur la base du mouvement (BRD) respiratoire enregistré du patient (P), dans lequel l'adaptation est effectuée dans la phase de scan du processus d'imagerie médicale,
- **caractérisé en ce que**, pendant la phase de scan, il est déterminé si le patient (P) retient sa respiration correctement et, dans le cas où le patient (P) ne retient pas sa respiration correctement, le processus de scan est reparamétrisé dynamiquement en augmentant le pas.

2. Procédé suivant la revendication 1, dans lequel l'adaptation est effectuée en outre dans au moins l'une des phases suivantes du processus d'imagerie médicale :
- la phase de pré-scan,
- la phase d'après-scan.

3. Procédé suivant la revendication 2, dans lequel, pendant la phase de pré-scan, l'adaptation comprend entraîner le patient (P) à respirer au mieux pendant un mode (SCM) de scan donné ou une tâche en prenant en compte un comportement (BRD) de respiration individuel du patient (P) pour le mode (SCM) de scan donné ou la tâche.

4. Procédé suivant la revendication 2 ou 3, dans lequel, pendant la phase de pré-scan l'adaptation comprend déterminer, en fonction du patient, des instructions (RI) de respiration temporisées de manière optimale individualisées.

5. Procédé suivant la revendication 4, dans lequel, pendant la phase de pré-scan, le point optimum du temps (STP) pour commencer le scan (SC) est déterminé sur la base d'une analyse d'une configuration détectée du comportement (BRD) respiratoire du patient (P).

6. Procédé suivant l'une quelconque des revendications 2 à 5, dans lequel, pendant la phase de pré-scan, le processus (SC) de scan est paramétrisé sur la base du comportement (BRD) respiratoire du patient (P).

7. Procédé suivant l'une quelconque des revendications 1 à 6, dans lequel, dans le cas où le patient (P) ne retient pas sa respiration correctement,
- une instruction (RI) est donnée pour rappeler au patient (P) de retenir sa respiration et le motiver.

8. Procédé suivant l'une quelconque des revendications 1 à 7, dans lequel, pendant la phase de scan, le processus de scan est interrompu, s'il a été détecté, sur la base du comportement (BRD) respiratoire enregistré, que les résultats du scan sont probablement insuffisants et qu'un re-scan ne peut pas être évité.

9. Procédé suivant l'une quelconque des revendications 1 à 7, dans lequel, pendant la phase, il est déterminé si le patient (P) maintient sa respiration correctement et, dans le cas où le patient (P) ne retient pas sa respiration correctement,
- le processus (SC) de scan est arrêté et une pause du scanning est effectué en une position z, où le patient (P) est autorisé à arrêter de retenir sa respiration,
- après la pause, le patient (P) reçoit instruction de retenir à nouveau sa respiration, et
- le processus (SC) de scan est continué en la position z, où le processus (SC) de scan est arrêté ou à une position z, où le patient (P) retient encore sa respiration correctement.

10. Procédé suivant l'une quelconque des revendications 2 à 9, dans lequel, pendant la phase d'après-scan, le comportement respiratoire individuel du patient (P) est analysé sur la base d'une courbe (BC) de respiration enregistrée et, dans le cas où il est détecté que le patient (P) ne suit pas les instructions (RI) de respiration correctement pendant la phase de scan et si l'on s'attend à de graves artefacts, un re-scan est recommandé.

11. Procédé suivant l'une quelconque des revendications 2 à 9, dans lequel, dans au moins l'une de la phase de pré-scan, de la phase de scan ou de la phase d'après-scan, il est déterminé automatiquement, sur la base du comportement (BRD) de respiration enregistré, comment adapter le processus d'imagerie médicale de scan au comportement (BRD) respiratoire enregistré.

12. Dispositif (60) d'adaptation, configuré pour effectuer le procédé suivant l'une quelconque des revendications 1 à 11 et comprenant :
- une unité (61) de contrôle pour enregistrer le mouvement (BRD) respiratoire du patient (P) en contrôlant le substitut respiratoire,
- une unité (62, 63, 64, 65, 66) d'adaptation pour adapter le processus d'imagerie médicale sur la base du mouvement (BRD) respiratoire enregistré du patient (P).

13. Système (1) d'imagerie médicale, le système (1) d'imagerie médicale étant un système CT et comprenant :
- une unité (2) de scan pour effectuer une imagerie médicale d'un patient (P),
- un dispositif (60) d'adaptation suivant la revendication 12 pour adapter l'imagerie médicale de l'unité (2) de scan à un mouvement (BRD) respiratoire enregistré du patient (P),
- un dispositif (RS) capteur pour acquérir des données de capteur concernant le mouvement (BRD) respiratoire du patient (P).

14. Produit de programme d'ordinateur ayant un programme d'ordinateur, qui peut être chargé directement dans un dispositif (13a) de mémoire d'un système (1) d'imagerie médicale, ayant des parties de programme pour effectuer tous les stades de l'une quelconque des revendications 1 à 11, lorsque le programme d'ordinateur est effectué dans le système (1) d'imagerie médicale.

15. Support, déchiffrable par ordinateur, sur lequel des parties de programme qui peuvent être déchiffrées et exécutées par une unité (13) informatique, sont mises en mémoire, afin d'effectuer tous les stades des procédés suivant l'une quelconque des revendications 1 à **11,** lorsque les parties du programme sont exécutées par l'unité (13) informatique.
